# EUROPEAN PATENT APPLICATION

(11) **EP 2 727 943 A1**
(43) Date of publication of application: **07.05.2014**
(21) Application number: 12007506.4
(22) Date of filing: 05.11.2012
(51) Int. Cl.: C07K 16/28, C07K 16/32, C07K 16/00

(54) **Trispecific antibodies against human EGFR, HER2 and HER3**

(71) Applicant: MAB Discovery GmbH, 82061 Neuried (DE)
(72) Inventor: Fischer, Stephan, 82362 Weilheim (DE); Renninger, Stephanie, 81379 München (DE)
(74) Representative: Schreiner, Siegfried

(57) **Abstract**

A monoclonal antibody, characterized in specifically binding to human EGFR, HER2 and HER3 is useful for the manufacture of a pharmaceutical composition and for the treatment of cancer, especially breast, colon, lung, or pancreatic cancer.

## Description

The present invention relates to trispecific antibodies against human EGFR, HER2 and HER3, compositions, methods for the production and uses thereof.

### Background of the invention

Human Epidermal growth factor receptor (EGFR, HER1), its sequence and functions are described by UniProt P00533 (EC=2.7.10.1), human HER2, its sequence and functions are described by UniProt P04626 (Receptor tyrosine-protein kinase erbB-2, EC=2.7.10.1), human HER3, its sequence and functions are described by UniProt P21860 (Receptor tyrosine-protein kinase erbB-3, EC=2.7.10.1). Fendry BM. et al., Cancer Res. 50 (1990 1550-1558 generated by immunization in mice with EGFR an anti-EGFR antibody which moderately binds also to p185HER2.Bostrom J. et al., Science 323 (2009) 1610-1614 generated a repertoire of Herceptin^{®} antibody variants with mutations in the light chain (LC). Fabs thereof were generated which bind also VEGF. Bostrom J. et al. PLOS & (2011) e17887 discusses the thermodynamic background of mulitispecificity of antibodies. Schaefer G. et al., Cancer Cell 20 (2011) 472 generated an antibody binding to HER3/EGFR by phage display. WO9964461 relates to bispecific antibodies against Apo2 and DR4. Immunization of mice was performed with mixed antigens. WO9939729 relates to the use of an HER2, HER3 and/or HER4 activating ligand which is a heregulin (HRG) polypeptide. WO2008027236 relates to a method of making a bispecific antibody against VEGF and HER2 by diversifying VL. WO2007146959 relates to dimeric and oligomeric structures of HER extracellular domains and HER/Fc molecules. WO2008140493 relates to anti-EGFR family member antibodies and heterogenic bispecific antibodies comprising anti-EGFR family member antibodies. WO2010129304 relates to methods for making heterogenic molecules, such as bispecific antibodies. WO2010108127 relates to a heterogenic bispecific antibody comprising an antigen-binding domain that specifically binds to EGFR and HER3 wherein the toxicity of the antibody is less than the toxicity of an EGFR antagonist. WO2011056997 relates to a method of affinity maturation of an antibody. WO2011060206 relates to an antibody against HER-3 and a combination thereof with other antibodies of the HER family. WO2011140254 relates to a combination of anti-EGFR antibodies. WO2012007167 relates to a heterogenic bispecific antibody against HER2 and LewisY.

### Summary of the Invention

The invention relates to trispecific monoclonal antibodies against human EGFR, HER2, and HER3 and methods for the generation of such antibodies. EGFR, HER2, and HER3 are therapeutic targets, and the invention provides also compositions, B cells, methods of use, and methods of production of the antibodies according to the invention.

The invention comprises a monoclonal antibody, characterized in specifically binding to EGFR, HER2 and HER3. Preferably the antibody is a rabbit monoclonal antibody.

Preferably the invention comprises also an antibody according to the invention, characterized in that it comprises at the C-terminal end of the VH region SEQ NO:49 or 50 and/or at the C terminal end of the VL region SEQ ID NO:51.

Preferably the invention also provides a heavy chain variable (VH) region of an antibody characterized in specifically binding to EGFR, HER2 and HER3, which is at least 90% identical to a VH region selected from the group consisting of VH regions of SEQ ID NO:1, 2, 3, 4, 5, and 6.

The invention also provides a light chain variable (VL) region of an antibody according to the invention, which is at least 90% identical to a VL region selected from the group consisting of VL regions of SEQ ID NO:7, 8, 9, 10, 11, and 12.

The antibody according to the invention is preferably characterized in that its VH region is at least 90% identical to a VH region of SEQ ID NO:1+n and its VL region is at least 90% identical to a VL region of SEQ ID NO:7+n, wherein n is a number selected from the group consisting of 0, 1, 2, 3, 4, and 5.

The invention also provides a VH region of an antibody according to the invention, selected from the group consisting of VH regions of SEQ ID NO:1, 2, 3, 4, 5, and 6.

The invention also provides a VL region of an antibody according to the invention, selected from the group consisting of VL regions of SEQ ID NO:7, 8, 9, 10, 11, and 12.

The antibody according to the invention is preferably characterized in comprising a VH region of SEQ ID NO:1+n and a VL region of SEQ ID NO:7+n, wherein n is a number selected from the group consisting of 0, 1, 2, 3, 4, and 5. Antibody 25 is characterized by said variable region sequences wherein n=0, antibody 37 by n=1, antibody 48 by n=2, antibody 49 by n=3, antibody 65 by n=4, and antibody 86 by n=5.

The antibody according to the invention is preferably characterized in comprising a VH region and a VL region comprising the respective CDR1, CDR2 and CDR3 regions of an antibody selected of the group consisting of antibodies 25, 37, 48, 49, 65 and 86.

The invention therefore provides a VH region of an antibody according to the invention, selected from the group consisting of VH regions comprising a CDR1H region of SEQ ID NO:13, a CDR2H region of SEQ ID NO:19 and aCDR3H region of SEQ ID NO:25; a CDR1H region of SEQ ID NO:14, a CDR2H region of SEQ ID NO:20 and aCDR3H region of SEQ ID NO:26; a CDR1H region of SEQ ID NO:15, a CDR2H region of SEQ ID NO:21 and aCDR3H region of SEQ ID NO:27; a CDR1H region of SEQ ID NO:16, a CDR2H region of SEQ ID NO:22 and aCDR3H region of SEQ ID NO:28; a CDR1H region of SEQ ID NO:17, a CDR2H region of SEQ ID NO:23 and aCDR3H region of SEQ ID NO:29; or a CDR1H region of SEQ ID NO:18, a CDR2H region of SEQ ID NO:24 and aCDR3H region of SEQ ID NO:30.

The invention therefore provides a VL region of an antibody according to the invention, selected from the group consisting of VL regions comprising a CDR1L region of SEQ ID NO:31, a CDR2L region of SEQ ID NO:37 and aCDR3L region of SEQ ID NO:43; a CDR1L region of SEQ ID NO:32, a CDR2L region of SEQ ID NO:38 and aCDR3L region of SEQ ID NO:44; a CDR1L region of SEQ ID NO:33, a CDR2L region of SEQ ID NO:39 and aCDR3L region of SEQ ID NO:45; a CDR1L region of SEQ ID NO:34, a CDR2L region of SEQ ID NO:40 and aCDR3L region of SEQ ID NO:46; a CDR1L region of SEQ ID NO:35, a CDR2L region of SEQ ID NO:41 and aCDR3L region of SEQ ID NO:47; or a CDR1L region of SEQ ID NO:36, a CDR2L region of SEQ ID NO:42 and aCDR3L region of SEQ ID NO:48.

The antibody according to the invention is preferably characterized in that the antibody according to the invention comprises a VH region comprising a CDR1H region of SEQ ID NO:13+n, a CDR2H region of SEQ ID NO:19+n and aCDR3H region of SEQ ID NO:25+n and a VL region comprising a CDR1H region of SEQ ID NO:31+n, a CDR2H region of SEQ ID NO:37+n and aCDR3H region of SEQ ID NO:43+n; wherein n is a number selected from the group of 0, 1, 2, 3, 4, and 5.

The antibody according to the invention is preferably characterized in being a humanized or chimeric version of said antibody. Preferably, the antibody according to the invention is an antibody comprising antigen binding sequences from a rabbit donor grafted to a heterologous non-human, human, or humanized sequence (e.g., framework and/or constant domain sequences). Preferably, an antibody of the invention has rabbit V regions or rabbit CDR regions and a human C region and/or framework. Preferably, the rabbit VL region or a human framework region comprising rabbit light chain CDRs is fused to a human kappa light chain constant region. Preferably, the rabbit VH region or a human framework region comprising rabbit heavy chain CDRs is fused to a human constant region, preferably IgG1.

The invention also provides a pharmaceutical composition characterized by comprising an antibody according to the invention. The invention also provides the use of an antibody according to the invention for the manufacture of a pharmaceutical composition. The invention also provides an antibody according to the invention for the treatment of cancer. The invention also provides an antibody according to the invention for the treatment of breast, colon, lung, or pancreatic cancer. The invention also provides the use of an antibody according to the invention for manufacture of a medicament for the treatment of cancer. The invention also provides the use of an antibody according to the invention for manufacture of a medicament for the treatment of breast, colon, lung, or pancreatic cancer. The invention also provides an antibody according to the invention for use in the treatment of cancer, preferably in the treatment breast, colon, lung, or pancreatic cancer.

The invention also provides a nucleic acid encoding an antibody according to the invention. The invention also provides an expression vector characterized in comprising a nucleic acid according to the invention for the expression of an antibody according to the invention in a prokaryotic or eukaryotic host cell. The invention also provides a prokaryotic or eukaryotic host cell comprising a nucleic acid according to the invention. The invention also provides a method of producing an antibody according to the invention characterized by expressing a nucleic acid according to the invention in a prokaryotic or eukaryotic host cell and recovering said antibody from said cell or the cell culture supernatant.

The invention also provides a method for inhibiting EGFR, HER2 and/or HER3 activity in a cell expressing EGFR, HER2 and/or HER3, comprising contacting the cell with an antibody according to the invention.

The invention also provides a method for the production of an antibody specifically binding to human EGFR, HER2, and HER3, characterized in performing in the order specified the steps of
i) immunizing a rabbit with an EGFR, HER2, and HER3 antigen,
ii) isolating B cells from said rabbit,
iii) isolating B cells from said B cells isolated in step ii) which bind to human EGFR, HER2, and/or HER3,
iv) isolating single B cells from said B cells isolated in step iii),
v) selecting one of said single B cells which comprises mRNA encoding a polypeptide comprising a VH region and/or a polypeptide comprising a VL region of an antibody which binds specifically to EGFR, HER2 and HER3, and
vi) producing the antibody expressed by said single B cell.

Preferably the EGFR, HER2, and/or HER3 antigen used for immunization is/are fusion polypeptides consisting of said antigen and a human Fc polypeptide. Preferably in step i) CFA is used as adjuvant. Preferably in step i) CFA and IFA are used together as adjuvants.

Preferably in step ii) B cells are isolated from the blood of the rabbit. B cells are isolated preferably as PBMCs and depleted from macrophages. The antigens used for isolating B cells in step iii) can be the EGFR, HER2, and HER3 proteins, fragments thereof, preferably the extracellular domain or parts thereof, cells presenting the antigens on their surface or the like.

Preferably in step iv) single B cells, secreting immunoglobulin, preferably IgG, are separated, preferably by FACS. Preferably the single B cell is then treated with a feeder cell before performing step v).

In step v) one of said antibody producing single rabbit B cells is selected. Preferably in step v) a single B cell is selected which comprises mRNA encoding a polypeptide comprising a VH region which is at least 90% identical to the VH region of an antibody selected from the group consisting of antibodies comprising a VH region of SEQ ID NO:1+n and/or mRNA encoding a polypeptide comprising a VL region which is at least 90% identical to the VL region of an antibody selected from the group consisting of antibodies comprising VL region of SEQ ID NO:7+n, wherein n is a number selected from the group consisting of 0, 1, 2, 3, 4, and 5.

Preferably in step v) a single B cell is selected which comprises mRNA encoding a VH region of an antibody specifically binding to EGFR, HER2 and HER3, comprising a VH region selected from the group consisting of VH regions of SEQ ID NO:1+n and mRNA encoding a VL region of an antibody specifically binding to EGFR, HER2 and HER3, comprising a VL region selected from the group consisting of VL regions of SEQ ID NO:7+n, wherein n is a number selected from the group of 0, 1, 2, 3, 4, and 5.

Preferably the antibody produced by the single B cell is tested, preferably by ELISA, whether it binds specifically to human EGFR, HER2, and HER3 and selected if it binds. Preferably the antibody is recombinantly produced based on its nucleic acid and/or polypeptide sequence.

The invention also comprises a rabbit B cell, characterized in comprising mRNA encoding a polypeptide comprising a VH region and/or encoding a polypeptide comprising a VL region of an antibody according to the invention. The invention also comprises a method for the production of an antibody according to the invention, characterized in isolating from a rabbit B cell comprising mRNA encoding a polypeptide comprising a VH region and/or encoding a polypeptide comprising a VL region of an antibody according to the invention an antibody and selecting said antibody if it binds specifically to EGFR, HER2 and HER3.

Preferably the anti- EGFR, HER2, and HER3 antibodies of the present invention are antagonistic antibodies.

### Detailed Description of the Invention

The term "rabbit" according to the invention means animals of the family Leporidae, preferably of genus Oryctolagus.

The term "antibody" encompasses the various forms of antibody structures including, but not being limited to, whole antibodies and antibody fragments. The antibody according to the invention is in its primary form produced by a B-cell of a rabbit and binds in this form specifically to human EGFR, HER2 and HER3. Therefore the antibody according to the invention binds specifically to human EGFR, HER2 and HER3 based on its antigen-binding portion, preferably its VH region comprising three VH CDRs and/or its VL region comprising three VL CDRs. The term "VL (or VH) region" has the same meaning as VL (or VH) domain. The antibody according to the invention is in its primary form a mature antibody, which may be different from a simple germline antibody. Without being bound by theory, it is believed that binding of the antigen to a germline antibody might lead to significant structural rearrangements, whereas the unbound state of a matured antibody might be closer to its bond state. Therefore the mature form of the antibody has probably a more rigid structure than the germline form. The germline antibody might be therefore more conformational flexible, resulting in a slower binding rate (see e.g. Wedemayer GJ et al., Science. 1997 Jun 13;276(5319):1665-9; Structural insights into the evolution of an antibody combining site). The presumably lower flexible structure of the mature antibody may improve the physicochemical properties of the antibody according to the invention, as being e.g. solubility or low aggregation, leading to improved therapeutic properties. The antibody according to the invention as identified from a rabbit B cell is an antibody having variable regions of natural origin. "Natural origin" means according to the invention, that such an antibody has variable regions which are identical in their amino acid sequences to the sequences of variable regions naturally occurring in rabbits. The antibody according to the invention can be further modified and is preferably a rabbit antibody, a humanized antibody, a chimeric antibody, a fragment thereof, or a further genetically engineered and recombinant produced antibody as long as the characteristic properties according to the invention are retained. The antibody can be bound to a further agent, e.g. as being an immunoconjugate.

The term "rabbit monoclonal antibody "means a monoclonal antibody produced by immunizing a rabbit and isolated from a B cell of said rabbit. Preferably the antibody is from a B cell of said rabbit. Preferably the antibody according to the invention is characterized in that it comprises at the C terminal end of the VH region SEQ NO.:49 or 50 and/or at the C terminal end of the VL region SEQ ID NO:51.

An "immunoconjugate" means an antibody conjugated to one or more cytotoxic agents, such as a chemotherapeutic agent, a drug, a growth inhibitory agent, a toxin, another antibody or a radioactive isotope.

"Antibody fragments" comprise a portion of a full length antibody, preferably the variable regions thereof, or at least the antigen binding site thereof. Examples of antibody fragments include diabodies, Fab fragments, and single-chain antibody molecules. scFv antibodies are, e.g., described in Huston, J.S., Methods in Enzymol. 203 (1991) 46-88.

The terms "monoclonal antibody" or "monoclonal antibody composition" as used herein refer to a preparation of antibody molecules of a single amino acid composition. The term "chimeric antibody" refers to a monoclonal antibody comprising a variable region, i.e., binding region, from rabbit and at least a portion of a constant region derived from a different source or species, usually prepared by recombinant DNA techniques. According to the invention chimeric antibodies comprising a rabbit variable region and a human constant region and humanized rabbit antibodies are especially preferred. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art (see, e.g., Morrison, S.L., et al, Proc. Natl. Acad. Sci. USA 81 (1984) 6851-6855; US 5,202,238 and US 5,204,244).

The term "humanized antibody" or "humanized version of an antibody" refers to antibodies in which a human variable region has been modified to comprise the CDRs of an antibody according to the invention. In a preferred embodiment, the CDRs of the VH and VL are grafted into the framework region of human antibody to prepare the "humanized antibody." See e.g. Riechmann, L., et al, Nature 332 (1988) 323-327; and Neuberger, M.S., et al, Nature 314 (1985) 268-270. The heavy and light chain variable framework regions can be derived from the same or different human antibody sequences. The human antibody sequences can be the sequences of naturally occurring human antibodies. Human heavy and light chain variable framework regions are listed e.g. in Lefranc, M.-P., Current Protocols in Immunology (2000) - Appendix IP A.1P.1-A.1P.37 and are accessible via IMGT, the international ImMunoGeneTics information system^{®} (http://imgt.cines.fr) or via http://vbase.mrc-cpe.cam.ac.uk.

The term "recombinant antibody", as used herein, is intended to include all antibodies according to the invention that are prepared by recombinant means, such as antibodies from a host cell such as a NS0 or CHO cell using a recombinant expression vector transfected into a host cell. Such recombinant human antibodies have variable and constant regions in a rearranged form.

The term "anti-EGFR, HER2, and HER3 antibody", as used herein, refer to an antibody which binds specifically to each of human EGFR, HER2, and HER3. For production of a trispecific antibody according to the invention at least the respective three antigens must be used and it is not possible to produce such an antibody by immunization with only one or two antigens.

The term "specifically binding ", as used herein, refer to binding of the antibody to the respective antigen, measured by ELISA, wherein said ELISA comprises coating EGFR, HER2, and HER3 protein respectively to a solid support, adding said antibody under conditions to allow the formation of an immune complex with the respective HER protein, detecting said immune complex by measuring the Optical Density values (OD) using a secondary antibody binding to an antibody according to the invention and using a peroxidase-mediated color development. Specific binding is found if at an antibody concentration of 300 µmol/l or more an OD value of 0.3 or more, preferably 1.0 or more, preferably 1.5 or more, preferably 2.0 or more preferably 1.0 or more (measured against same solvent without antibody at 450nm) is found. Preferably the antibody according to the invention binds to EGFR and HER2 in a ratio of 1:10 to 10:1, to EGFR and HER3 in a ratio of 1:10 to 10:1 and to HER2 and HER3 in a ratio of 1:10 to 10:1 measured by ELISA wherein said ELISA comprises: coating EGFR, HER2, and HER3 protein respectively to a solid support, adding said antibody under conditions to allow the formation of an immune complex with the respective HER protein, detecting said immune complex by measuring the Optical Density using a secondary antibody binding to said antibody and a peroxidase-mediated color development, and calculating said binding ratios using said Optical Density values. The antibodies according to the invention do not bind specifically to other human antigens like human Fc, human IL12R, human HER4, HGFR, Notch-1, CD44, IGF-1R, P-Cadherin, EpoR, or DLL4. Therefore for these and other such antigens OD values will be 0.1 or lower (e.g. 0.0).

The antibody according to the invention comprises a VH region and a VL region or parts thereof, which are together sufficient for the specific binding to EGFR, HER2, and HER3 according to the invention. Therefore the antibody according to the invention is different to antibodies which are sometimes called "bispecific, multispecific etc. antibodies" in the state of the art and which comprise according to the state of the art at least a VH and a VL region from a first antibody and a VH and a VL region from a second antibody wherein each of those antibodies binds to a different antigen or epitope or which comprise several single-domains of nanobodies (such antibodies of the state of the art are described e.g. by Kontermann R., MAbs. 2012 Mar 1;4(2); and Caravella J, Lugovskoy, A.Curr Opin Chem Biol. 2010 Aug;14(4):520-8).

The terms "EGFR, HER2, and HER3" or "antigens" or "HER proteins", as used herein, refer to Human Epidermal growth factor receptor (EGFR, HER1), its sequence and functions are described by UniProt P00533 (EC=2.7.10.1), human HER2, its sequence and functions are described by UniProt P04626 (Receptor tyrosine-protein kinase erbB-2, EC=2.7.10.1), and human HER3, its sequence and functions are described by UniProt P21860 (Receptor tyrosine-protein kinase erbB-3, EC=2.7.10.1).

The "variable region (or domain) of an antibody according to the invention" (variable region of a light chain (VL), variable region of a heavy chain (VH)) as used herein denotes each of the pair of light and heavy chain regions which are involved directly in binding the antibody to the antigen. The variable light and heavy chain regions have the same general structure and each region comprises four framework (FR) regions whose sequences are widely conserved, connected by three complementary determining regions, CDRs. The term "antigen-binding portion of an antibody" when used herein refer to the amino acid residues of an antibody which are responsible for antigen-binding. The antigen-binding portion of an antibody comprises preferably amino acid residues from the "complementary determining regions" or "CDRs". The CDR sequences are defined according to Kabat et al, Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR or CDR of the variable region. For example, a heavy chain variable region may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence. The variable domain of the heavy chain of an antibody according to the invention is composed of a single immunoglobulin domain and is about 110 to 120 amino acids long. The variable domain of the light chain of an antibody according to the invention is composed of a single immunoglobulin domain and is about 110 to 120 amino acids long.

In one embodiment the antibody according to the invention comprises a Fc part derived from human origin and preferably all other parts of the human constant regions. As used herein the term "Fc part derived from human origin" denotes a Fc part which is either a Fc part of a human antibody of the subclass IgG1, IgG2, IgG3 or IgG4, e.g. a Fc part from human IgG1 subclass, a mutated Fc part from human IgG1 subclass (preferably with a mutation on L234A + L235A), a Fc part from human IgG4 subclass or a mutated Fc part from human IgG4 subclass (preferably with a mutation on S228P). In one embodiment the antibody according to the invention is of human IgG1 subclass. Human constant chains are well known in the state of the art and e.g. described by Kabat, E.A., (see e.g. Johnson, G. and Wu, T.T., Nucleic Acids Res. 28 (2000) 214-218).

In one embodiment the antibody according to the invention comprises a heavy chain variable region (VH) sequence having at least 90%, 91%, 92%>, 93%>, 94%, 95%, 96%, 97%, 98%, 99%, or 100% sequence identity to an amino acid sequence selected from the group of SEQ ID NO:1+n wherein n is a number from 0 to 5. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, whereby the antibody retains the ability to bind specifically according to the invention to EGFR,HER2 and HER3. In certain embodiments, a total of 1 to 10 amino acids have been substituted, inserted and/or deleted in each of SEQ ID NO:1+n. In certain embodiments, substitutions, insertions, or deletions occur in regions outside the CDRs (i.e., in the FRs).

In one embodiment the antibody according to the invention comprises a light chain variable region (VL) having at least 90%, 91%, 92%, 93%, 94%, 95%, 96%, 97%, 98%, 99%, or 100% sequence identity to the amino acid sequence of SEQ ID NO:7+n, wherein n is a number from 0 to 5. In certain embodiments, a VL sequence having at least 90%, 91%, 92%, 93%, 94%, 95%, 96%, 97%, 98%, or 99%o identity contains substitutions (e.g., conservative substitutions), insertions, or deletions relative to the reference sequence, whereby the antibody retains the ability to bind specifically to EGFR,HER2 and HER3. In certain embodiments, a total of 1 to 10 amino acids have been substituted, inserted and/or deleted in SEQ ID NO:7+n . In certain embodiments, the substitutions, insertions, or deletions occur in regions outside the CDRs (i.e., in the FRs).

Identity or homology with respect to the sequence is defined herein as the percentage of amino acid residues in the candidate sequence that are identical with the parent sequence, after aligning the sequences and introducing gaps, if necessary, to achieve the maximum percent sequence identity. None of N-terminal, C-terminal, or internal extensions, deletions, or insertions into the antibody sequence shall be construed as affecting sequence identity or homology. The variant retains the ability to bind specifically to EGFR, HER2 and HER3 and preferably has properties, which are superior to those of the parent antibody. For example, the variant may have improved binding to the targets. An exemplary "parent" antibody comprises the CDR regions of antibody 25 and is preferably used for the preparation of the variant. Preferably, the parent antibody has a human framework region and, if present, has human antibody constant domains. For example, the parent antibody may be a humanized antibody. An "affinity matured" antibody is one with one or more alterations in one or more CDRs thereof which result in an improvement in the affinity of the antibody for antigen, compared to a parent antibody which does not possess those alteration(s). Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al., Proc Nat. Acad. Sci, USA 91 :3809-3813 (1994); Schier et al., Gene 169: 147-155 (1995); Yelton et al., J. Immunol. 1 55 : 1994-2004 (1995); Jackson et al., J. Immunol. 1 54(7):3310-9 (1995); and Hawkins et al., J. Mol. Biol. 226:889-896 (1992) and WO2010108127. "Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) softwa re

The number "n" according to the invention is meant to be identical for the same antibody, its heavy and light chains, its variable regions and CDR regions. That means that e.g. for antibody 25 (n=0) the VH region comprises SEQ ID NO:1, the VL region comprises SEQ ID NO: 7, and the heavy chain CDR1H sequence comprises SEQ ID NO:13. Sequence numbers and values of "n" of variable chains and CDRs of antibodies according to the invention are shown in table 1.

**Table 1**

| **Antibody** | **SEQ ID NO. of antibody region** | | | | | | | | |
|---|---|---|---|---|---|---|---|---|---|
| | VH | VL | CDR1H | CDR2H | CDR3H | CDRL1 | CDRL2 | CDRL3 | n |
| 25 | 1 | 7 | 13 | 19 | 25 | 31 | 37 | 43 | 0 |
| 37 | 2 | 8 | 14 | 20 | 26 | 32 | 38 | 44 | 1 |
| 48 | 3 | 9 | 15 | 21 | 27 | 33 | 39 | 45 | 2 |
| 49 | 4 | 10 | 16 | 22 | 28 | 34 | 40 | 46 | 3 |
| 65 | 5 | 11 | 17 | 23 | 29 | 35 | 41 | 47 | 4 |
| 86 | 6 | 12 | 18 | 24 | 30 | 36 | 42 | 48 | 5 |

Cell-mediated effector functions like ADCC of antibodies according to the invention can be enhanced by engineering their oligosaccharide component as described in Umana, P., et al, Nature Biotechnol. 17 (1999) 176-180, and US 6,602,684, WO 2005/044859, WO 2004/065540, WO2007/031875. The term "antibody-dependent cellular cytotoxicity (ADCC)" refers to lysis of human target cells by an antibody according to the invention in the presence of effector cells.

The antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains of an antibody according to the invention or fragments thereof are inserted into expression vectors by standard methods. Expression is performed in appropriate prokaryotic or eukaryotic host cells, such as CHO cells, NSO cells, SP2/0 cells, HEK293 cells, COS cells, yeast, or E. coli cells, and the antibody is recovered from the cells (from the supernatant or after cells lysis). Recombinant production of antibodies is well-known in the state of the art and described, for example, in the review articles of Makrides, S.C., Protein Expr. Purif. 17 (1999) 183-202; Geisse, S., et al, Protein Expr. Purif. 8 (1996) 271-282; Kaufman, R.J., Mol. Biotechnol. 16 (2000) 151-161; Werner, R.G., Drug Res. 48 (1998) 870-880. The antibodies may be present in whole cells, in a cell lysate, or in a partially purified, or pure form. Purification is performed in order to eliminate other cellular components or other contaminants, e.g., other cellular nucleic acids or proteins, by standard techniques, including, column chromatography and others well known in the art (see Ausubel, F., et al, ed. Current Protocols in Molecular Biology, Greene Publishing and Wiley Interscience, New York (1987)). Expression in NSO cells is described by, e.g., Barnes, L.M., et al, Cytotechnology 32 (2000) 109-123; Barnes, L.M., et al, Biotech. Bioeng. 73 (2001) 261-270. Transient expression is described by, e.g.,Durocher, Y., et al, Nucl. Acids. Res. 30 (2002) E9. Cloning of variable domains is described by Orlandi, R., et al, Proc. Natl. Acad. Sci. USA 86 (1989) 3833- 3837; Carter, P., et al, Proc. Natl. Acad. Sci. USA 89 (1992) 4285-4289; Norderhaug, L., et al, J. lmmunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199. Monoclonal antibodies are suitably separated from the culture medium by conventional immunoglobulin purification procedures such as, for example, protein A-Sepharose, hydroxylapatite chromatography or affinity chromatography.

DNA and RNA encoding the monoclonal antibodies are sequenced using conventional procedures. RT PCR is preferably used.

Antibodies obtained from said cell lines are preferred embodiments of the invention. Amino acid sequence variants of an antibody are prepared by introducing nucleotide changes into the antibody encoding DNA, or by peptide synthesis. Any cysteine residue not involved in maintaining the proper conformation of the antibody may also be substituted, generally with serine, to improve the oxidative stability of the molecule and to prevent aberrant crosslinking. Conversely, cysteine bond(s) may be added to the antibody to improve its stability (particularly where the antibody is an antibody fragment such as an Fv fragment).

The heavy and light chain variable regions according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The invention also provides a pharmaceutical composition comprising an antibody according to the invention. Another aspect of the invention is the use of an antibody according to the invention for the manufacture of a pharmaceutical composition. A further aspect of the invention is a method for the manufacture of a pharmaceutical composition comprising an antibody according to the invention. In another aspect, the present invention provides a composition, e.g. a pharmaceutical composition, containing an antibody according to the present invention, formulated together with a pharmaceutical carrier.

Furthermore the antibodies according to the invention are especially useful for the treatment of diseases associated with a dysregulation of HER pathways, e.g. cancer. For this the antibody according to the invention can be investigated in a respective mouse tumor model e.g. according to Krupke DM; Begley DA; Sundberg JP; Bult CJ; Eppig JT, The Mouse Tumor Biology database., Nat Rev Cancer 2008 Jun;8(6):459-65. Therefore one aspect of the invention is a pharmaceutical composition for the treatment of cancer.

Another aspect of the invention is an antibody according to the invention for the treatment of cancer.

Another aspect of the invention is the use of an antibody according to the invention for the manufacture of a medicament for the treatment of cancer.

Another aspect of the invention is a method of treatment of a patient suffering from cancer by administering an antibody according to the invention to said patient in the need of such treatment.

As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

The term "cancer" as used herein may be, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. Preferably such cancer is a breast cancer, colon cancer, lung cancer, or pancreatic cancer. Preferably such cancers are further characterized by EGFR, HER2 and/or HER3 expression or overexpression.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin. Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art. Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The method according to the invention comprises in summary the steps of immunization, B cell isolation, enrichment of B cells, isolation of single B cells, preferably co-cultivation with feeder cells, selection of a single B cell which comprises respective mRNA, and production of the antibody according to the invention. Such methods are mentioned for the production of monospecific antibodies e.g. in WO2011147903, WO2007003041, WO2008045140, WO2004106377, EP1255780, and EP1633787.

### Immunization

Immunization can be performed according to the methods known of the state of the art, e.g. by using DNA of the antigens or fragments thereof, complete protein antigens or fragments thereof, antigen expressing cells. Preferably the EGFR, HER2, and/or HER3 antigen is/are fusion polypeptides consisting of said antigen and a human Fc polypeptide. Preferably immunization in step i) is repeated at least three times and appropriately up to six times during 90 days (if an antibody according to the invention is identified already after e.g. the fourth immunization, further immunizations are not necessary). Preferably complete Freund's adjuvant (CFA) or CFA and incomplete Freund's adjuvant (IFA) is (are) used as adjuvant.

### B cell isolation

The B-cells are isolated from the rabbit, preferably from the blood of the rabbit. The B-cells are isolated up to 8 days, preferably 6 to 8 days, after 3rd to 6th immunization. Preferably PBMCs are isolated and depleted from macrophages (see e.g. EP0488470) and used as B cells in step iii). Isolation of B cells can be for example also performed by labeling non-B cells with non B cell markers, e.g. anti CD2, CD14, CD16, CD36, CD43, and CD235a antibodies and separating the labeled non B cells from non-labeled B cells.

### Enrichment of B cells

Antigen specific B cells are preferably isolated (enriched) in step iii) by treating the B cells with the EGFR, HER2 and/or HER3 antigens or a cell expressing one or more of the respective antigens. The antigens used for enrichment must not be all three antigens used for immunization. It is also sufficient to use in this step only one or two of the antigens. Preferably the antigens and the cell expressing the antigens are used in immobilized manner, so that the antigen specific B cells can be separated easily. Such methods are e.g. described in Kodituwakko AP et al., Immunol. Cell Biol. (2003) 81, 163-170 and EP0488470.

### Isolation of single B cells

Isolation of single rabbit B cells in step iv) is preferably performed by FACS. Preferably an anti-rabbit IgG is used for FACS selection. Such selected single B cells are antibody producing B cells.

### Co-cultivation with feeder cells

Preferably the antigen producing B cells are co-cultivated with feeder cells after step iv) and before the selection step v) is performed. This increases the amount of antibody in the cell supernant and facilitates analysis and selection of secreted rabbit antibodies specifically binding to human EGFR, HER2, and HER3 (see e.g. Zubler, R.H., et al., Eur. J. immune). 14 (1984) 357-63, Wen L. et al., Eur. J. Immunol. 17 (1987) 887-92, Hoffmann P et al., J Immunol. Methods 1996;196(1):85-91, Roy A. et al., J Hematother. Stem Cell Res. 2001; 10(6):873-80, Dlu A. et al., Proc. Natl. Acad. Sci. USA Vol. 84, pp. 9140-9144, 1987, and EP0488470).

### Selection of a single B cell which comprises mRNA

Selection of a single B cell which comprises mRNA encoding polypeptides comprising a heavy and light chain variable region of an antibody according to the invention can be performed, preferably after co-cultivated with feeder cells, by analyzing the cell supernatant for secreted rabbit antibodies specifically binding to human EGFR, HER2, and HER3. Analysis is preferably performed by ELISA. Immunoglobulin sequences can be then recovered from the selected single human B cell e.g. according to de Wildt RM, Hoet RM. Methods Mol. Biol. 2002; 178:121-31 and analyzed e.g. by RT PCR.

The production of an antibody according to the invention, expressed by a single B cell, can be performed by recombinant means.

Techniques and procedures described or referenced herein are for example, the widely utilized methodologies described in Sambrook et al., Molecular Cloning: A Laboratory Manual 3rd. edition (2001) Cold Spring Harbor Laboratory Press, Cold Spring Harbor, N.Y. CURRENT PROTOCOLS IN MOLECULAR BIOLOGY (F. M. Ausubel, et al. eds., (2003)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)).

The following examples and sequences are provided to aid the understanding of the present invention. It is understood that modifications can be made in the procedures set forth without departing from the spirit of the invention.

### Examples

### Example 1

### Immunization of rabbits (scheme 1)

Three recombinant human Fc-chimera proteins fused with the extracellular domains of the tyrosine kinase receptors Her1, Her2 and Her3 were used as immunogen mixture. Two different immunization schemes, scheme 1 and scheme 2, were explored.

For the immunization according to scheme 1, three New Zealand White (NZW) rabbits were immunized by injecting 1ml of immunogen mixture in each of the animals at day 0, 7, 14, 28, 56 and 84. Proteins were diluted in PBS, pooled in equimolar amounts and mixed 1:1 (v/v) with complete Freund's adjuvant (CFA) before use. A final concentration of 100µg of each immunogen (300µg in total) was used per animal for the 1st immunization and for the 2nd, 3rd, 4th, 5th and 6th immunization 50µg of each immunogen (150µg in total) and per animal was used. Blood samples were collected in tubes, coated with EDTA five, six and seven days post-immunization after the 3rd, 4th, 5th and 6th Immunization.

### Example 2

### Immunization of rabbits (scheme 2)

For the immunization according to scheme 2, each of the three NZW rabbits were immunized subcutaneously with 1ml of immunogen mixture at day 0, 7, 14, 28, 42 and 56. For the first injection, proteins were diluted in PBS, pooled in equimolar amounts and mixed 1:1 (v/v) with CFA before use. A final concentration of 100µg of each Immunogen (300µg in total) per animal was used for the 1st immunization. For the 2nd, 3rd, 4th, 5th and 6th immunization, proteins were diluted in PBS, pooled in equimolar amounts and mixed 1:1 (v/v) with incomplete Freund's adjuvant (IFA) before use. 50µg of each Immunogen (150µg in total) was used per animal. Blood samples were collected in tubes, coated with EDTA five, six and seven days post-immunization after the 3rd, 4th, 5th and 6th Immunization at intervals of 2 weeks.

### Example 3

### 3.1 Immunogen Coating/Cell Preparation

The three fusion-proteins used for immunization were coated onto a surface of a cell-culture 6-well plate with a concentration of 8µg in PBS/10cm² and incubated. Alternatively plates were seeded with a cell line BT-474 (DSMZ ACC 64) expressing the three tyrosine kinase receptors EGFR, Her2 and Her3 on their cell surface. One day before use cells were seeded in DMEM+5%FCS at a density leading to about 90% confluence after 24h.

### 3.2 Isolation of peripheral blood mononuclear cells from rabbits

PBMCs were isolated from whole blood of immunized rabbits. The blood was diluted 1:1 with PBS and layered on Lympholyte^{®} according to the manufacturer's instructions (Cedarlane, CL5120). Peripheral blood mononuclear cells (PBMC) were separated from erythrocytes by density gradient centrifugation (800xg, 20min, RT). Cells were removed from the interface, washed twice with PBS (800xg, 10min) and suspended in RPMI 1640 based cell culture medium.

### 3.3 Monocyte depletion

PBMCs were incubated in cell culture medium on plastic. Unbound lymphocytes were collected after incubation time.

### 3.4 Enrichment of antigen specific cells

Antigen specific lymphocytes were enriched on Immunogen coated microtiter plates or directly on BT-474 cells (see 3.1.). Lymphocytes were washed twice with PBS to remove unspecific cells and subsequently incubated with 750µl Trypsin per 10cm² culture surface for 7-10min. Detached cells were collected in cell culture medium for further steps.

### 3.5 Single-cell sorting of Immunoglobulin G-secreting lymphocytes

PBMCs/lymphocytes were stained with a FITC (Fluorescein Isothiocyanate Isomer 1) conjugated goat anti-rabbit IgG antibody, Abd Serotec, STAR121F). A flow cytometric analysis and single-cell sorting was performed with a FACS cytometer. Single positive lymphocytes were sorted directly to 200µl cell culture medium covering 3,0x10⁶ irradiated EL-4 B5 feeder cells (L. Wen et al. Eur. J. Immunol. 17 (1987) 887-892). The cell culture medium described above was supplemented with 5% activated T-cell macrophage supernatant from rabbits (MicroCoat). Co-cultivation medium was supplemented with 2x10⁻⁰⁶ g/ml SAC (Staphylococcus Aureus Cowan) solution. After co-cultivation of B-cells and feeder cells for 7 days supernatants were transferred for antibody detection and cells were harvested in 100µl RNA isolation buffer (Qiagen, RLT).

### 3.6 Screening for Immunoglobulin's via enzyme-linked immunosorbent assay

Secreted rabbit antibodies were detected by analyzing the supernatant via a biotinylated capturing antibody (anti-rabbit IgG antibody produced in goat) with a final concentration of 1µg/ml PBS+0,5%BSA+0,05%Tween^{®}20, coated on streptavidin microtiter plates and a horse radish peroxidase coupled anti-rabbit IgG detection antibody with a final concentration of 1:7500. Washing steps were performed by using PBS+0.1%Tween^{®}20. 3,3',5,5'-Tetramethylbenzidine (TMB) was used as substrate and HCl to stop the enzymatic reaction.

### 3.7 Determination of HER specific antibodies in B-cell Supernatants

Microtiter plates were coated with EGFR, HER2, HER3 protein (recombinant Fc chimeric conjugates of human EGFR, HER2, HER3 or IL12 Rß1). After a blocking process, specific antibodies from B-cell supernatants bind to the targets and are then detected by a POD-labeled anti-rabbit IgG antibody. The IL12R binding was used as a counter-screen. The HER proteins were tagged with a linker, huFc and His (HER1 does not have a His-tag) like the IL12R protein. Antibodies which bind to the tag were positive in both assays, whereas antigen specific antibodies just bound to the HER proteins and not to IL12R.

12.5µL 0.5µg/mL HER protein in PBS was transferred to a microtiter plate incubated and washed 3x with Wash Buffer. 90µL Block Buffer was added to each well, incubated and washed. 12.5µL Standard Antibody (Anti-EGF-Receptor, ERBB2 Rabbit anti-Human Monoclonal (extracellular domain) (SP3) Antibody; ERBB3/HER3 (N-terminal) antibody; start concentration 2µg/ml; IL-12Rbeta1 antibody start concentration 500ng/ml) in 1:2 dilutions or sample diluted in ELISA buffer was added, incubated and washed. 12.5µl 1:5000 POD-Antibody (Anti-rabbit IgG, peroxidase-linked species-specific Fab2 fragment (from donkey) (ECL); assay dilution: 1:5000) in Elisa Buffer was added, incubated and washed. 15µl TMB was added and 15µl HCl was added after sufficient development. Absorbance (Optical Density O.D.) was read at 450nm/620nm. Six antibodies according to the invention were identified according to example 1 to 3. Results are shown in table 2.
ELISA Buffer: PBS, 0.5% BSA, 0.05% Tween^{®}20
Wash Buffer: PBS, 0.1% Tween^{®}20
Block Buffer: PBS, 2% BSA, 0.05% Tween^{®}20

**Table 2**

| **Antibody** | **Optical density (OD) measured for antigen** | | | | **IgG concentration ng/ml** |
|---|---|---|---|---|---|
| | EGFR | HER2 | HER3 | IL12R | |
| 25 | 3.3 | 2.4 | 2.6 | 0.0 | 1148.9 |
| 37 | 2.9 | 2.7 | 2.7 | 0.0 | 719.9 |
| 48 | 1.2 | 1.2 | 1.1 | 0.0 | 81.8 |
| 49 | 2.5 | 0.4 | 0.6 | 0.0 | 377.5 |
| 65 | 2.5 | 2.9 | 1.9 | 0.0 | 425.7 |
| 86 | 1.1 | 1.8 | 0.8 | 0.0 | 1500.0 |

### Example 4

### Immunization of rats (comparison)

Immunization was performed according to example2 (immunization scheme 2) with Wistar rats.

Overall 441 anti-huHer1, 774 anti-huHer2 and 299 anti-huHer3 antibodies were isolated after four blood sample collection steps from each rat (10 weeks after immunization start) by a multiple immunization strategy. No trispecific antibodies were identified.

## Claims

1. A monoclonal antibody, **characterized in** specifically binding to human EGFR, HER2 and HER3.

2. An antibody according to claim 1, **characterized in that** its VH region is at least 90% identical to a VH region of SEQ ID NO:1+n, its VL region is at least 90% identical to a VL region of SEQ ID NO:7+n, wherein n is a number selected from the group of 0, 1, 2, 3, 4, and

3. An antibody according to claim 1, **characterized in** comprising a VH region of SEQ ID NO:1 and a VL region of SEQ ID NO:7, a VH region of SEQ ID NO:2 and a VL region of SEQ ID NO:8, a VH region of SEQ ID NO:3 and a VL region of SEQ ID NO:9, a VH region of SEQ ID NO:4 and a VL region of SEQ ID NO:10, a VH region of SEQ ID NO:5 and a VL region of SEQ ID NO:11, or a VH region of SEQ ID NO:6 and a VL region of SEQ ID NO:12.

4. An antibody according to claim 1, **characterized in that** the antibody comprises a VH region comprising a CDR1H region of SEQ ID NO:13+n, a CDR2H region of SEQ ID NO:19+n and aCDR3H region of SEQ ID NO:25+n and a VL region comprising a CDR1H region of SEQ ID NO:31+n, a CDR2H region of SEQ ID NO:37+n and aCDR3H region of SEQ ID NO:43+n; wherein n is a number selected from the group of 0, 1, 2, 3, 4, and 5.

5. An antibody according to any one of claims 1 to 4, **characterized in** being a monoclonal rabbit antibody.

6. Pharmaceutical composition **characterized by** comprising an antibody according to any one of claims 1 to 5.

7. An antibody according to any one of claims 1 to 5 for use in the treatment of cancer.

8. Nucleic acid encoding an antibody of any one of claims 1 to 5.

9. A method of producing a monoclonal antibody that specifically binds to EGFR, HER2 and HER3 **characterized by** expressing a nucleic acid according to claim 8 in a prokaryotic or eukaryotic host cell and recovering said antibody from said cell or the cell culture supernatant.

10. A method for the production of a monoclonal antibody specifically binding to human EGFR, HER2, and HER3, **characterized in** performing in the order specified the steps of
i) immunizing a rabbit with an EGFR, HER2, and HER3 antigen,
ii) isolating B cells from said rabbit,
iii) isolating B cells from said B cells isolated in step ii) which bind to human EGFR, HER2, and/or HER3,
iv) isolating single B cells from said B cells isolated in step iii),
v) selecting one of said single B cells which comprises mRNA encoding a polypeptide comprising a VH region and/or a polypeptide comprising a VL region of an antibody which binds specifically to EGFR, HER2 and HER3, and
vi) producing the antibody expressed by said single B cell.

11. A rabbit B cell, **characterized in** comprising mRNA encoding a polypeptide comprising a VH region and/or mRNA encoding a polypeptide comprising a VL region of an antibody according to any one of claims 1 to 5.

12. A method for the production of an antibody according to any one of claims 1 to 5, **characterized in** isolating from a rabbit B cell comprising mRNA encoding a polypeptide comprising a VH region and/or mRNA encoding a polypeptide comprising a VL region of said antibody, expressing recombinantly said antibody and selecting said antibody if it binds specifically to EGFR, HER2 and HER3.


SEQUENCE LISTING

<110> Mab Discovery GmbH

<120> Trispecific Antibodies against EGFR, HER2 and HER3

<130> MD1201

<160> 51

<170> BiSSAP 1.2

<210> 1
<211> 116
<212> PRT
<213> Oryctolagus

<400> 1
Gln Ser Leu Glu Glu Ser Gly Gly Asp Leu Val Lys Pro Gly Ala Ser
1               5                   10                  15
Leu Thr Leu Ile Cys Thr Ala Ser Gly Phe Asp Leu Asn Thr Asn Tyr
            20                  25                  30
Trp Ile Cys Trp Val Arg Gln Ala Pro Gly Lys Gly Leu Glu Trp Ile
        35                  40                  45
Ala Cys Ile Asp Ser Gly Tyr Gly Asp Ile Tyr Tyr Ala Thr Trp Ala
    50                  55                  60
Lys Gly Arg Phe Thr Ile Ser Lys Ala Ser Ser Thr Thr Val Thr Leu
65                  70                  75                  80
Gln Met Thr Ser Leu Thr Ala Ala Asp Thr Ala Thr Tyr Phe Cys Ala
                85                  90                  95
Arg Glu Ile Ile Phe His Phe Asn Leu Trp Gly Pro Gly Thr Leu Val
            100                 105                 110
Thr Val Ser Ser
        115

<210> 2
<211> 116
<212> PRT
<213> Oryctolagus

<400> 2
Gln Ser Leu Glu Glu Ser Gly Gly Asp Leu Val Gln Pro Glu Gly Ser
1               5                   10                  15
Leu Thr Leu Thr Cys Lys Ala Ser Gly Leu Asp Phe Ser Ser Ser Tyr
            20                  25                  30
Trp Ile Cys Trp Val Arg Gln Ala Pro Gly Lys Gly Leu Glu Trp Ile
        35                  40                  45

Ala Cys Ile Asp Thr Gly Tyr Gly Asp Ile Tyr Tyr Ala Ser Trp Ala
    50                  55                  60
Lys Gly Arg Phe Thr Met Ser Lys Thr Ser Ser Thr Thr Val Thr Leu
65                  70                  75                  80
Gln Met Thr Ser Leu Thr Ala Ala Asp Thr Ala Thr Tyr Phe Cys Ala
                85                  90                  95
Arg Glu Lys Ile Phe His Phe Asn Leu Trp Gly Pro Gly Thr Leu Val
            100                 105                 110
Thr Val Ser Ser
        115

<210> 3
<211> 116
<212> PRT
<213> Oryctolagus

<400> 3
Gln Ser Leu Glu Glu Ser Gly Gly Asp Leu Val Lys Pro Gly Ala Ser
1               5                   10                  15
Leu Thr Leu Thr Cys Thr Ala Ser Gly Phe Asp Leu Asn Ser Asp Tyr
            20                  25                  30
Trp Ile Cys Trp Val Arg Gln Ala Pro Gly Lys Gly Leu Glu Trp Ile
        35                  40                  45
Ala Cys Ile Asp Thr Gly Tyr Gly Asp Ile Tyr Tyr Ala Ser Trp Ala
    50                  55                  60
Lys Gly Arg Phe Thr Ile Ser Lys Thr Ser Ser Thr Thr Val Thr Leu
65                  70                  75                  80
Gln Met Thr Ser Leu Thr Ala Ala Asp Thr Ala Thr Tyr Phe Cys Ala
                85                  90                  95
Arg Glu Ile Ile Phe His Phe Asn Leu Trp Gly Pro Gly Thr Leu Val
            100                 105                 110
Thr Val Ser Ser
        115

<210> 4
<211> 116
<212> PRT
<213> Oryctolagus

<400> 4
Gln Ser Leu Glu Glu Ser Gly Gly Asp Leu Val Lys Pro Gly Ala Ser
1               5                   10                  15
Leu Thr Leu Thr Cys Thr Ala Ser Gly Phe Asp Leu Asn Ser Asn Tyr
            20                  25                  30
Trp Ile Cys Trp Val Arg Gln Ala Pro Gly Lys Gly Leu Glu Trp Ile
        35                  40                  45
Ala Cys Ile Asp Thr Gly Tyr Gly Asp Ile Tyr Tyr Ala Ser Trp Ala
    50                  55                  60

Lys Gly Arg Phe Thr Ile Ser Lys Thr Ser Ser Thr Thr Val Thr Leu
65                  70                  75                  80
Gln Met Thr Ser Leu Thr Ala Ala Asp Thr Ala Thr Tyr Phe Cys Ala
                85                  90                  95
Arg Glu Ile Ile Phe His Phe Asn Leu Trp Gly Pro Gly Thr Leu Val
            100                 105                 110
Thr Val Ser Ser
        115

<210> 5
<211> 117
<212> PRT
<213> Oryctolagus

<400> 5
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Asn Asn Tyr Tyr
            20                  25                  30
Met Thr Trp Val Arg Gln Ala Pro Gly Lys Gly Leu Glu Trp Ile Gly
        35                  40                  45
Val Ile Ser Gly Ser Ala Thr Thr Tyr Tyr Ala Ser Trp Ala Lys Gly
    50                  55                  60
Arg Phe Thr Ile Ser Gln Thr Ser Thr Thr Val Asp Leu Arg Ile Thr
65                  70                  75                  80
Ser Pro Thr Thr Glu Asp Thr Ala Thr Tyr Phe Cys Ala Arg Ser Leu
                85                  90                  95
Thr Ala Asp Ile Ile Asp Tyr Tyr Asn Ile Trp Gly Pro Gly Thr Leu
            100                 105                 110
Val Thr Val Ser Leu
        115

<210> 6
<211> 115
<212> PRT
<213> Oryctolagus

<400> 6
Gln Glu Gln Leu Lys Glu Ser Gly Gly Gly Leu Val Thr Pro Gly Gly
1               5                   10                  15
Thr Leu Thr Leu Thr Cys Thr Val Ser Gly Ile Asp Leu Ser Asn Tyr
            20                  25                  30
Ala Met Val Trp Val Arg Gln Ala Pro Gly Lys Gly Leu Glu Trp Ile
        35                  40                  45
Gly Ile Ile Tyr Thr Thr Ala Ser Thr Thr Tyr Ala Asn Trp Ala Lys
    50                  55                  60
Gly Arg Phe Thr Ile Ser Lys Thr Ser Thr Thr Val Asp Leu Lys Ile
65                  70                  75                  80

Thr Ser Pro Thr Thr Glu Asp Thr Ala Thr Tyr Phe Cys Val Arg Arg
                85                  90                  95
Ser Gly Trp Asp Gly Phe Asp Pro Trp Gly Pro Gly Thr Leu Val Thr
            100                 105                 110
Val Ser Ser
        115

<210> 7
<211> 111
<212> PRT
<213> Oryctolagus

<400> 7
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Arg Ser Tyr
            20                  25                  30
Leu Ala Trp Tyr Gln Gln Lys Pro Gly Gln Pro Pro Lys Val Leu Ile
        35                  40                  45
Tyr Lys Ala Ser Thr Leu Ala Ser Gly Val Ser Ser Arg Phe Lys Gly
    50                  55                  60
Ser Gly Ser Gly Thr Gln Phe Thr Leu Thr Ile Ser Asp Leu Glu Cys
65                  70                  75                  80
Ala Asp Ala Ala Thr Tyr Tyr Cys Gln Gly Gly Val Glu Phe Asn Asn
                85                  90                  95
Tyr Leu Tyr Asn Ser Phe Gly Gly Gly Thr Glu Val Val Val Lys
            100                 105                 110

<210> 8
<211> 111
<212> PRT
<213> Oryctolagus

<400> 8
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Lys Asn Tyr
            20                  25                  30
Leu Ser Trp Tyr Gln Gln Lys Pro Gly Gln Pro Pro Lys Val Leu Leu
        35                  40                  45
Tyr Lys Ala Ser Thr Leu Ala Ser Gly Val Ser Ser Arg Phe Lys Gly
    50                  55                  60
Ser Gly Ser Gly Thr Gln Phe Thr Leu Thr Ile Ser Asp Leu Glu Cys
65                  70                  75                  80
Ala Asp Ala Ala Thr Tyr Tyr Cys Gln Gly Gly Val Glu Val Asn Asn
                85                  90                  95
Tyr Leu Tyr Asn Pro Phe Gly Gly Gly Thr Glu Val Val Val Lys
            100                 105                 110

<210> 9
<211> 111
<212> PRT
<213> Oryctolagus

<400> 9
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Arg Ser Tyr
            20                  25                  30
Leu Ala Trp Tyr Gln Gln Lys Pro Gly Gln Pro Pro Lys Val Leu Ile
        35                  40                  45
Tyr Lys Ala Ser Thr Leu Ala Ser Gly Val Ser Ser Arg Phe Lys Gly
    50                  55                  60
Ser Gly Ser Gly Thr Gln Phe Thr Leu Thr Ile Ser Asp Leu Glu Cys
65                  70                  75                  80
Ala Asp Ala Ala Thr Tyr Tyr Cys Gln Gly Gly Val Glu Leu Asn Asn
                85                  90                  95
Tyr Leu Tyr Asn Pro Phe Gly Gly Gly Thr Glu Val Val Val Lys
            100                 105                 110

<210> 10
<211> 111
<212> PRT
<213> Oryctolagus

<400> 10
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Ser Ser Tyr
            20                  25                  30
Leu Ala Trp Tyr Gln Gln Lys Pro Gly Gln Pro Pro Lys Val Leu Ile
        35                  40                  45
Tyr Lys Ala Ser Thr Leu Ala Ser Gly Val Ser Ser Arg Phe Lys Gly
    50                  55                  60
Ser Gly Ser Gly Thr Gln Phe Thr Leu Thr Ile Ser Asp Leu Glu Cys
65                  70                  75                  80
Ala Asp Ala Ala Thr Tyr Tyr Cys Gln Gly Gly Val Glu Val Asn Asn
                85                  90                  95
Tyr Leu Tyr Asn Pro Phe Gly Gly Gly Thr Glu Val Val Val Lys
            100                 105                 110

<210> 11
<211> 110
<212> PRT
<213> Oryctolagus

<400> 11
Asp Val Val Met Thr Gln Thr Pro Ala Ser Val Ser Glu Pro Val Gly
1               5                   10                  15
Gly Thr Val Thr Ile Lys Cys Gln Ala Ser Glu Asp Ile Asn Ala Leu
            20                  25                  30
Leu Ala Trp Tyr Gln Gln Lys Pro Gly Gln Pro Pro Lys Leu Leu Ile
        35                  40                  45
Tyr Val Ala Ser Tyr Leu Gln Ser Gly Val Pro Ser Arg Phe Lys Gly
    50                  55                  60
Ser Gly Ser Gly Thr Glu Tyr Thr Leu Thr Ile Ser Gly Val Gln Cys
65                  70                  75                  80
Ala Asp Ala Ala Thr Tyr Tyr Cys Gln Ser Ala Tyr Asp Ser Ser Gly
                85                  90                  95
Tyr Val Gly Ala Phe Gly Gly Gly Thr Glu Val Val Val Lys
            100                 105                 110

<210> 12
<211> 110
<212> PRT
<213> Oryctolagus

<400> 12
Ala Gln Val Leu Thr Gln Thr Pro Ala Ser Val Ser Ala Ala Val Gly
1               5                   10                  15
Gly Thr Val Thr Ile Asn Cys Gln Ala Ser Glu Ser Ile Ser Ser Tyr
            20                  25                  30
Leu Asn Trp Tyr Gln Gln Lys Pro Gly Gln Pro Pro Lys Arg Leu Ile
        35                  40                  45
Tyr Arg Thr Ser Thr Leu Ala Ser Gly Val Pro Ser Arg Phe Lys Gly
    50                  55                  60
Ser Gly Ser Gly Thr Asp Phe Thr Leu Thr Ile Ser Gly Val Glu Cys
65                  70                  75                  80
Ala Asp Ala Ala Thr Tyr Tyr Cys Gln Gln Asp Trp Ser Ser Ser Asp
                85                  90                  95
Val Glu Asn Val Phe Gly Gly Gly Thr Glu Val Val Val Lys
            100                 105                 110

<210> 13
<211> 6
<212> PRT
<213> Oryctolagus

<400> 13
Thr Asn Tyr Trp Ile Cys
1               5

<210> 14

<211> 6
<212> PRT
<213> Oryctolagus

<400> 14
Ser Ser Tyr Trp Ile Cys
1               5

<210> 15
<211> 6
<212> PRT
<213> Oryctolagus

<400> 15
Ser Asp Tyr Trp Ile Cys
1               5

<210> 16
<211> 6
<212> PRT
<213> Oryctolagus

<400> 16
Ser Asn Tyr Trp Ile Cys
1               5

<210> 17
<211> 5
<212> PRT
<213> Oryctolagus

<400> 17
Asn Tyr Tyr Met Thr
1               5

<210> 18
<211> 5
<212> PRT
<213> Oryctolagus

<400> 18
Asn Tyr Ala Met Val
1               5

<210> 19

<211> 17
<212> PRT
<213> Oryctolagus

<400> 19
Cys Ile Asp Ser Gly Tyr Gly Asp Ile Tyr Tyr Ala Thr Trp Ala Lys
1               5                   10                  15
Gly

<210> 20
<211> 17
<212> PRT
<213> Oryctolagus

<400> 20
Cys Ile Asp Thr Gly Tyr Gly Asp Ile Tyr Tyr Ala Ser Trp Ala Lys
1               5                   10                  15
Gly

<210> 21
<211> 17
<212> PRT
<213> Oryctolagus

<400> 21
Cys Ile Asp Thr Gly Tyr Gly Asp Ile Tyr Tyr Ala Ser Trp Ala Lys
1               5                   10                  15
Gly

<210> 22
<211> 17
<212> PRT
<213> Oryctolagus

<400> 22
Cys Ile Asp Thr Gly Tyr Gly Asp Ile Tyr Tyr Ala Ser Trp Ala Lys
1               5                   10                  15
Gly

<210> 23
<211> 16
<212> PRT
<213> Oryctolagus

<400> 23
Val Ile Ser Gly Ser Ala Thr Thr Tyr Tyr Ala Ser Trp Ala Lys Gly
1               5                   10                  15

<210> 24
<211> 16
<212> PRT
<213> Oryctolagus

<400> 24
Ile Ile Tyr Thr Thr Ala Ser Thr Thr Tyr Ala Asn Trp Ala Lys Gly
1               5                   10                  15

<210> 25
<211> 8
<212> PRT
<213> Oryctolagus

<400> 25
Glu Ile Ile Phe His Phe Asn Leu
1               5

<210> 26
<211> 8
<212> PRT
<213> Oryctolagus

<400> 26
Glu Lys Ile Phe His Phe Asn Leu
1               5

<210> 27
<211> 8
<212> PRT
<213> Oryctolagus

<400> 27
Glu Ile Ile Phe His Phe Asn Leu
1               5

<210> 28
<211> 8
<212> PRT
<213> Oryctolagus

<400> 28
Glu Ile Ile Phe His Phe Asn Leu
1               5

<210> 29
<211> 12
<212> PRT
<213> Oryctolagus

<400> 29
Ser Leu Thr Ala Asp Ile Ile Asp Tyr Tyr Asn Ile
1               5                   10

<210> 30
<211> 9
<212> PRT
<213> Oryctolagus

<400> 30
Arg Ser Gly Trp Asp Gly Phe Asp Pro
1               5

<210> 31
<211> 11
<212> PRT
<213> Oryctolagus

<400> 31
Gln Ala Ser Gln Ser Ile Arg Ser Tyr Leu Ala
1               5                   10

<210> 32
<211> 11
<212> PRT
<213> Oryctolagus

<400> 32
Gln Ala Ser Gln Ser Ile Lys Asn Tyr Leu Ser
1               5                   10

<210> 33
<211> 11
<212> PRT
<213> Oryctolagus

<400> 33
Gln Ala Ser Gln Ser Ile Arg Ser Tyr Leu Ala
1               5                   10

<210> 34
<211> 11
<212> PRT
<213> Oryctolagus

<400> 34
Gln Ala Ser Gln Ser Ile Ser Ser Tyr Leu Ala
1               5                   10

<210> 35
<211> 11
<212> PRT
<213> Oryctolagus

<400> 35
Gln Ala Ser Glu Asp Ile Asn Ala Leu Leu Ala
1               5                   10

<210> 36
<211> 11
<212> PRT
<213> Oryctolagus

<400> 36
Gln Ala Ser Glu Ser Ile Ser Ser Tyr Leu Asn
1               5                   10

<210> 37
<211> 7
<212> PRT
<213> Oryctolagus

<400> 37
Lys Ala Ser Thr Leu Ala Ser
1               5

<210> 38
<211> 7
<212> PRT
<213> Oryctolagus

<400> 38
Lys Ala Ser Thr Leu Ala Ser
1               5

<210> 39
<211> 7
<212> PRT
<213> Oryctolagus

<400> 39
Lys Ala Ser Thr Leu Ala Ser
1               5

<210> 40
<211> 7
<212> PRT
<213> Oryctolagus

<400> 40
Lys Ala Ser Thr Leu Ala Ser
1               5

<210> 41
<211> 7
<212> PRT
<213> Oryctolagus

<400> 41
Val Ala Ser Tyr Leu Gln Ser
1               5

<210> 42
<211> 7
<212> PRT
<213> Oryctolagus

<400> 42
Arg Thr Ser Thr Leu Ala Ser
1               5

<210> 43
<211> 13
<212> PRT
<213> Oryctolagus

<400> 43
Gln Gly Gly Val Glu Phe Asn Asn Tyr Leu Tyr Asn Ser
1               5                   10

<210> 44
<211> 13
<212> PRT
<213> Oryctolagus

<400> 44
Gln Gly Gly Val Glu Val Asn Asn Tyr Leu Tyr Asn Pro
1               5                   10

<210> 45
<211> 13
<212> PRT
<213> Oryctolagus

<400> 45
Gln Gly Gly Val Glu Leu Asn Asn Tyr Leu Tyr Asn Pro
1               5                   10

<210> 46
<211> 13
<212> PRT
<213> Oryctolagus

<400> 46
Gln Gly Gly Val Glu Val Asn Asn Tyr Leu Tyr Asn Pro
1               5                   10

<210> 47
<211> 12
<212> PRT
<213> Oryctolagus

<400> 47
Gln Ser Ala Tyr Asp Ser Ser Gly Tyr Val Gly Ala
1               5                   10

<210> 48
<211> 12
<212> PRT
<213> Oryctolagus

<400> 48
Gln Gln Asp Trp Ser Ser Ser Asp Val Glu Asn Val
1               5                   10

<210> 49
<211> 11
<212> PRT
<213> Oryctolagus

<400> 49
Trp Gly Pro Gly Thr Leu Val Thr Val Ser Ser
1               5                   10

<210> 50
<211> 11
<212> PRT
<213> Oryctolagus

<400> 50
Trp Gly Pro Gly Thr Leu Val Thr Val Ser Leu
1               5                   10

<210> 51
<211> 10
<212> PRT
<213> Oryctolagus

<400> 51
Phe Gly Gly Gly Thr Glu Val Val Val Lys
1               5                   10